# EUROPÄISCHE PATENTSCHRIFT

(11) **EP 0 890 649 B1**
(45) Veröffentlichungstag und Bekanntmachung des Hinweises auf die Patenterteilung: **03.03.2004**
(21) Anmeldenummer: 98109999.7
(22) Anmeldetag: 02.06.1998
(51) Int. Cl.: C12P 13/04, C12P 41/00, C07C 229/48, C07C 321/06, C07C 233/63

(54) **Verfahren zur Herstellung von acylierten Aminosäureestern und Racematspaltung von Aminosäureestern durch Enzym-katalysierte Acylierung**
Process for the preparation of acylated amino acid esters and resolving racemic mixtures of amino acid esters by enzyme-catalysed acylation
Procédé pour la préparation des esters d'acides aminés acylés et séparation des racemates des esters d'acides aminés par acylation enzymatique

(30) Priorität: 30.06.1997 DE 19727517
(43) Veröffentlichungstag der Anmeldung: 13.01.1999
(73) Patentinhaber: BASF AKTIENGESELLSCHAFT, 67056 Ludwigshafen (DE)
(72) Erfinder: Stürmer, Rainer, Dr., 67127 Rödersheim-Gronau (DE); Ditrich, Klaus, Dr., 67161 Gönnheim (DE); Siegel, Wolfgang, Dr., 67117 Limburgerhof (DE)

(56) Entgegenhaltungen:
- F.FACHE ET AL.: "A Catalytic Stereo- and Chemo-Selective Method for the Reduction of Substituted Aromatics" TETRAHEDRON LETT., Bd. 36, Nr. 6, 1995, Seiten 885-888, XP004028696
- MUI MUI SIM ET AL.: "Combinatorial Synthesis of 2-Thioxo-4-dihydropyrimidinones." J. ORG. CHEM., Bd. 62, Nr. 26, 1997, Seiten 9358-9360, XP002079156
- G. BERNATH ET AL.: "Stereochemical Studies." TETRAHEDRON, Bd. 43, Nr. 19, 1987, Seiten 4359-4366, XP002079157
- L. T. KANERVA ET AL.: "Approach to Highly Enantiopure beta-Amino Acid Esters by Using Lipase Catalysis in Organic Media." TETRAHEDRON: ASYMMETRY, Bd. 7, Nr. 6, 1996, Seiten 1705-1716, XP004047657
- V. M. SANCHEZ ET AL.: "Candida antarctica lipase catalyzed resolution of ethyl (+,-)-3-aminobutyrate." TETRAHEDRON: ASYMMETRY, Bd. 8, Nr. 1, 1997, Seiten 37-40, XP004015173
- B. ORSAT ET AL.: "Homocarbonates as Substrates for the Enantioselective Enzymatic Protection of Amines." J. AM. CHEM. SOC., Bd. 118, 1996, Seiten 712-713, XP002079158
- G. ASENSIO ET AL.: "Enzyme-Mediated Enantioselective Acylation of Secondary Amines in Organic Solvents." TETRAHEDRON LETT., Bd. 32, Nr. 33, 1991, Seiten 4197-4198, XP002000856

## Beschreibung

Die vorliegende Erfindung betrifft in Anspruch 1 ein Verfahren zur Herstellung von acylierten Aminosäureestern sowie ein neues Verfahren zur Herstellung von optisch aktiven Aminosäureestern aus racemischen Aminosäureestern mit einem Carbonsäurealkylester als Acylierungsmittel, dessen Säurekomponente ein Halogen-, Stickstoff-, Sauerstoff- oder Schwefelatom in Nachbarschaft des Carbonylkohlenstoffatoms trägt und dessen Alkoholkomponente einen unsubstituierten, verzweigt- oder geradkettigen C₁-C₁₀-Alkylrest darstellt, in Gegenwart einer Hydrolase, ausgewählt aus der Gruppe Amidase, Protease, Esterase und Lipase und anschließende Trennung des einen enantioselektiv acylierten Aminosäureesters vom nicht acylierten anderen Enantiomer des Aminosäureesters.

Die Racematspaltung primärer und sekundärer Amine durch Umsetzung mit einem Carbonsäureester in Gegenwart einer Hydrolase wird in WO 95/08636 und WO 96/23894 sowie in einem Übersichtsartikel von Balkenhohl et al. in J. prakt. Chem. 339 (1997) 381-384 beschrieben. Als bevorzugte Amine werden dort primäre Arylalkylamine und heteroatomsubstituierte Amine genannt. Es findet sich jedoch in allen drei Schriften kein Hinweis auf die Verwendbarkeit von Aminosäureestern.

Asensiso et al. (Tetrahedron Letters, 1991, 4197 - 4198) beschreiben die Lipase-vermittelte enantioselektive Acylierung cyclischer 1,2- bzw. 1,3-Aminoalkohole in organischen Lösemitteln. Als Acylierungsmittel dient dabei Ethylacetat. Es wird eine starke Abhängigkeit des Reaktionsverlaufs von der Art des eingesetzten Enzymes, der Reaktionszeit und -temperatur sowie vom Substrat festgestellt.

Den Einsatz von Homocarbonaten als Substrate für die enantioselektive enzymatische Schützung von Aminen beschreiben Orsat et al. (J. Am. Chem. Soc., 1996, 712 - 713). Die danach zugänglichen Carbamate werden bei sehr langen Reaktionszeiten in moderaten Ausbeuten und Enantiomerenüberschüssen erhalten.

Kanerva et al. (Tetrahdedron: Asymmetrie, 1996, 1705 - 1716) beschreiben einen Lipase-katalysierten Zugang zu enantiomerenangereicherten β-Aminosäureestern. Dabei werden die Ethylester verschiedener alicyclischer β-Aminosäuren in Gegenwart von Lipase SP 526 bzw. Lipase PS mit aktivierten Acylierungsmitteln umgesetzt. Als aktivierte Ester dienen verschiedene 2,2,2-Trifluoracetate. Eine überraschend günstige Beeinflussung des Reaktionsverlaufs bezüglich Reaktionszeit, Ausbeute und Enantiomerenüberschuß wurde beim Übergang zu einem doppelt aktivierten Acylierungsmittel (ClCH₂CO₂CH₂CF₃) beobachtet.

Sänchez et al. (Tetrahedron: Asymmetry, 1997, 37-40) beschreiben die enantioselektive Acylierung von 3-Aminobuttersäureethylester mit Ethylacetat unter Katalyse von Candida Antarctica Lipase (SP 435). Dabei benötigen sie jedoch für die Katalyse enorm große Enzymmengen (50 Gew.-% bezogen auf Substrat), so daß ein darauf aufbauendes Verfahren nicht wirtschaftlich ist. Außerdem ist das

Verfahren zu wenig enantioselektiv. Die erreichten Selektivitäten liegen nur im Bereich von E = 70-90.

Es bestand daher die Aufgabe, ein Verfahren zur enzymkatalysierten Racematspaltung von Aminosäureestern bereitzustellen, das eine hohe Enantioselektivität gewährleistet, in einem weiten Bereich von Reaktionsbedingungen eingesetzt werden kann und dabei mit möglichst geringen Mengen an Katalysator auskommt.

Demgemäß wurde gefunden, daß ein Verfahren zur Racematspaltung von Aminosäuren durch Umsetzung mit einem Carbonsäureester als Acylierungsmittel unter spezifischer Katalyse mit einer Hydrolase, ausgewählt aus der Gruppe Amidase, Protease, Esterase und Lipase und anschließende Trennung des einen, enantioselektiv acylierten Aminosäureesters vom nicht acylierten anderen Enantiomeren des Aminosäureesters dann besonders gut funktioniert, wenn die Säurekomponente des als Acylierungsmittel verwendeten Carbonsäureesters ein elektronenreiches Heteroatom, ausgewählt aus der Gruppe, die von Halogen-, Stickstoff-, Sauerstoff- und Schwefelatomen gebildet wird, in Nachbarschaft, d.h. in alpha-, betaoder gamma-Position des Carbonylkohlenstoffatoms trägt.

Weiterhin wurde ein Verfahren zur Herstellung von acylierten Aminosäureestern durch Umsetzung der Aminosäureester mit einem, als Acylierungsmittel verwendeten Carbonsäurealkylester unter spezifischer Katalyse mit einer Hydrolase, ausgewählt aus der Gruppe Amidase, Protease, Esterase und Lipase gefunden, das dadurch gekennzeichnet ist, daß die Säurekomponente des Esters ein Halogen-, Stickstoff-, Sauerstoff- oder Schwefelatom in Nachbarschaft des Carbonylkohlenstoffatoms trägt, und dass die Alkoholkomponente einen unsubstituierten, verzweigt- oder geradkettigen C₁-C₁₀-Alkylrest darstellt.

Die für das erfindungsgemäße Verfahren als Acylierungsmittel geeigneten Carbonsäureester sind solche, die in der Säurekomponente des Esters ein elektronenreiches Heteroatom in Nachbarschaft zum Carbonylkohlenstoff tragen.

Das Heteroatom muß über mindestens ein freies Elektronenpaar verfügen. Es kann ein Halogen, insbesondere Chlor- oder Fluor-, ein Stickstoff-, Sauerstoff- oder ein Schwefelatom sein.

Es soll sich in der Nachbarschaft zum Carbonylkohlenstoff befinden. Darunter ist die Bindung des Heteroatoms an ein Kohlenstoffatom in alpha-, beta- oder gamma-Position zum Carbonylkohlenstoff gemeint. Bevorzugt sind solche Säurekomponenten des Esters, bei denen das Heteroatom an das alpha C-Atom gebunden ist. Als Heteroatom ist Sauerstoff bevorzugt.

Das Heteroatom kann gegebenenfalls mit weiteren Gruppen, z.B. Alkyl- oder Arylgruppen, bevorzugt Alkylgruppen verknüpft sein. Ist das Heteroatom beispielsweise Sauerstoff, so liegt eine Ethergruppe vor.

Als Alkoholkomponente des Esters können für das erfindungsgemäße Verfahren unsubstituierte verzweigte und unverzweigte C₁- bis C₁₀-Alkohole, bevorzugt C₁- bis C₆-Alkohole, besonderes bevorzugt C₂- bis C₅-Alkohole verwendet werden.

Besonders geeignete Carbonsäureester sind solche der Formel III, 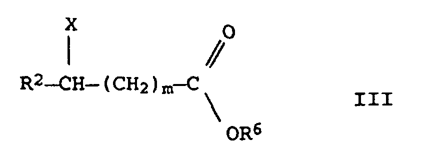 worin
- R²: H, C₁-C₁₀-Alkyl,
- X: Halogen, OR³, SR³, NR³R⁴,
- R³: H, C₁-C₁₀-Alkyl,
Aryl, gegebenenfalls substituiert durch NH₂, OH, C₁-C₄-Alkyl, C₁-C₄-Alkoxy oder Halogen,
- R⁴: H, C₁-C₁₀-Alkyl,
Aryl, gegebenenfalls substituiert durch NH₂, OH, C₁-C₄-Alkyl, C₁-C₄-Alkoxy oder Halogen,
- R⁶: C₁-C₁₀-Alkyl,
- m: 0, 1 oder 2
bedeuten. Unter diesen sind die C₁-C₄-Alkylester der C₁-C₄-Alkoxyessigsäuren, wie Methoxyessigsäureethylester oder Methoxyessigsäureisopropylester, besonders bevorzugt.

Als Hydrolasen können in dem erfindungsgemäßen Verfahren eine Vielzahl von Enzymen eingesetzt werden. Bevorzugt werden Amidasen, Proteasen, Esterasen und Lipasen, insbesondere Lipasen verwendet. Als Lipasen sind vor allem mikrobielle Lipasen gut geeignet, die beispielsweise aus Hefen oder Bakterien isolierbar sind. Besonders gut geeignet sind Lipasen aus Pseudomonas, z. B. Amano P oder die Lipase aus Pseudomonas spec. DSM 8246. Weitere besonders gut geeignete Hydrolasen sind die von Novo Nordisk (Enzyme Toolbox) kommerziell erhältlichen Enzyme, insbesondere die Lipasen SP 523, SP 524, SP 525, SP 526 und Novozym® 435. Diese Enzyme sind mikrobielle Lipasen, die aus Hefen wie Candida antarctica herstellbar sind.

Das verwendete Enzym kann in nativer oder in immobilisierter Form eingesetzt werden. Besonders gut eignet sich das immobilisierte Enzym Novozym® 435.

Die erfindungsgemäßen Verfahren können unter Verwendung von Lösungsmitteln oder bei flüssigen Aminosäureestern auch lösungsmittelfrei durchgeführt werden.

Als Lösungsmittel sind generell organische Lösungsmittel geeignet. Besonders gut verläuft die Reaktion in Ethern; beispielsweise in MTBE (= Methyl-tert.butylether) oder THF (= Tetrahydrofuran), oder in Kohlenwasserstoffen wie Hexan, Cyclohexan, Toluol oder halogenierten Kohlenwasserstoffen wie Methylenchlorid. Bei schwerlöslichen Edukten kann noch Propylencarbonat, Acetonitril oder Dioxan zugesetzt werden.

Die Umsetzung des Carbonsäureesters mit dem racemischen Aminosäureester unter Enzymkatalyse wird üblicherweise bei Temperaturen zwischen 20 und 50°C, insbesondere zwischen 25 und 35°C ausgeführt. Die Reaktionszeiten dafür betragen je nach Aminosäureester 1 bis 48, bevorzugt 4 bis 24 Stunden. Sekundäre Aminosäureester benötigen in der Regel längere Reaktionszeiten als primäre Aminosäureester. Die geringere Reaktivität sekundärer Aminosäureester kann auch durch eine gegenüber primären Aminosäureestern erhöhte Menge an Katalysator ausgeglichen werden.

Die Konzentration an eingesetztem Aminosäureester liegt im Bereich von 5 bis 50 Gew.-%, bevorzugt im Bereich von 10 bis 30 Gew.-%, bezogen auf die Gesamtmenge des Reaktionsgemisches.

Pro Mol umzusetzender Aminosäureester werden 1 bis 3 Mol, bevorzugt 1 bis 2 Mol, besonders bevorzugt 1 bis 1,5 Mol Carbonsäureester zugesetzt.

Die zuzusetzende Menge an Enzym hängt von der Art der Hydrolase und der Aktivität der Enzympräparation ab. Die für die Reaktion optimale Enzymmenge kann leicht durch einfache Vorversuche ermittelt werden.

Der Reaktionsverlauf läßt sich leicht mit üblichen Methoden beispielsweise mittels Gaschromatographie verfolgen. Im Falle der Racematspaltung beendet man die Reaktion sinnvollerweise bei einem Umsatz von 50% des racemischen Aminosäureesters. Dies geschieht in der Regel durch Entfernen des Katalysators aus dem Reaktionsraum, beispielsweise durch Abfiltrieren des Enzyms.

Durch die enantioselektive Umsetzung des racemischen Aminosäureesters mit dem als Acylierungsmittel verwendeten Carbonsäureester entsteht aus dem einen Enantiomer das entsprechend acylierte Produkt (Amid), während das andere Enantiomer unverändert bleibt.

Das nun vorliegende Gemisch aus Aminosäureester und dem N-acylierten Derivat läßt sich mit üblichen Methoden leicht trennen. Gut geeignet zur Trennung des Gemisches sind beispielsweise Extraktions- oder Destillationsverfahren.

Das erfindungsgemäße Verfahren eignet sich zur Acylierung sowie zur Racematspaltung von α-, β- und γ-Aminosäureestern. Besonders gut verläuft die Racematspaltung bei β-Aminosäureestern, insbesondere bei cyclischen β-Aminosäureestern der folgenden Formel IV, 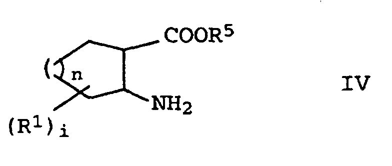 in der R⁵ für C₁-C₁₀-Alkyl, bevorzugt C₁-C₆-Alkyl, besonders bevorzugt C₂-C₅-Alkyl sowie für, gegebenenfalls durch NH₂, OH, C₁-C₄-Alkyl, C₁-C₄-Alkoxy oder Halogen substituiertes Aryl, bevorzugt Phenyl oder Benzyl steht und in der n Werte von 1 bis 3 einnehmen kann.

Der Carbocyclus kann gegebenenfalls durch R¹ substituiert sein, wobei R¹ C₁-C₁₀-Alkyl, bevorzugt C₁-C₆-Alkyl, besonders bevorzugt C₂-C₅-Alkyl, C₁-C₄-Alkoxy, OH, NH₂, Halogen, bevorzugt Cl bedeuten kann sowie darüberhinaus für Aryl, gegebenenfalls substituiert durch NH₂, OH, C₁-C₄-Alkyl, C₁-C₄-Alkoxy oder Halogen, bevorzugt Phenyl oder Benzyl steht. Die Anzahl der Substituenten R¹ wird durch den Index i bestimmt und kann 0 bis 4 betragen.

Die Erfindung eignet sich auch zur Herstellung von optisch aktiven Aminosäureestern aus racemischen Aminosäureestern, indem man
a) einen racemischen Aminosäureester mit einem Carbonsäurealkylester als Acylierungsmittel, dessen Säurekomponente ein Fluor-, Stickstoff-, Sauerstoff- und Schwefelatom in Nachbarschaft des Carbonylkohlenstoffatoms trägt und dessen Alkoholkomponente einen unsubstituierten, verzweigt- oder geradkettigen C₁-C₁₀-Alkylrest darstellt, in Gegenwart einer Hydrolase enantioselektiv acyliert,
b) das Gemisch aus optisch aktivem Aminosäureester und optisch aktivem N-Acyl-Aminosäureester trennt und somit ein Enantiomer des Aminosäureesters erhält,
c) gewünschtenfalls aus dem N-Acyl-Aminosäureester das andere Enantiomere des Aminosäureesters durch Amidspaltung gewinnt.

Das erfindungsgemäße Verfahren läßt sich noch ökonomischer gestalten, wenn man nach Abtrennung des gewünschten Enantiomers das verbliebene, nicht gewünschte Enantiomer racemisiert und erneut in das Verfahren einsetzt. So läßt sich beispielsweise ein nicht gewünschter acylierter Aminosäureester in Gegenwart von nicht-nucleophilen Basen wie z.B. Kalium-tert.butylat racemisieren und durch eine starke Säure (Mineralsäure) in den freien racemischen Aminosäureester überführen. Durch diese Rückführung wird es möglich, insgesamt mehr als 50% des gewünschten Enantiomers aus dem racemischen Amin zu erhalten.

Die erfindungsgemäßen Verfahren eignen sich nicht nur als Herstellverfahren zur Produktion optisch aktiver Aminosäureester, sondern können auch Bestandteil von komplizierten chemischen Mehrstufensynthesen, beispielsweise bei der Herstellung von Arzneiwirkstoffen oder Pflanzenschutzmitteln, sein.

Beispielsweise können gemäß dem erfindungsgemäßen Verfahren optisch aktive Aminosäureester der Formel I, 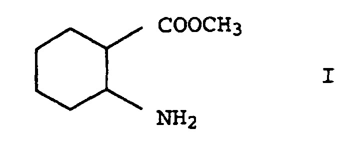 in der die NH₂-Gruppe und der Methoxycarbonylrest in cis- oder bevorzugt in trans-Stellung zueinander stehen, eingesetzt werden. Durch das erfindungsgemäße Verfahren erhält man optisch aktive N-Acyl-Aminosäureester der Formel II 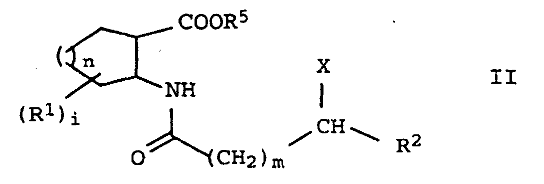 in der die Reste folgende Bedeutung haben:
- R¹: C₁-C₁₀-Alkyl,
C₁-C₄-Alkoxy, OH, NH₂, Halogen,
Aryl, gegebenenfalls substituiert durch NH₂, OH, C₁-C₄-Alkyl, C₁-C₄-Alkoxy oder Halogen
- R²: H, C₁-C₁₀-Alkyl
- X: Halogen, OR³, SR³, NR³R⁴,
- R³: H, C₁-C₁₀-Alkyl,
Aryl, gegebenenfalls substituiert durch NH₂, OH, C₁-C₄-Alkyl, C₁-C₄-Alkoxy oder Halogen
- R⁴: H, C₁-C₁₀-Alkyl,
Aryl, gegebenenfalls substituiert durch NH₂, OH, C₁-C₄-Alkyl, C₁-C₄-Alkoxy oder Halogen
- R⁵: C₁-C₁₀-Alkyl,
Aryl, gegebenenfalls substituiert durch NH₂, OH, C₁-C₄-Alkyl, C₁-C₄-Alkoxy oder Halogen
- i: 0 bis 4
- m: 0, 1 oder 2
- n: 1 bis 3
und in der die Acylaminogruppe und der COOR⁵-Rest sowohl in cisals auch in trans-Stellung zueinander stehen können.

Bevorzugte Reste für X sind Chlor oder OR³, wobei R³ für C₁-C₄-Alkyl steht. Für R⁵ steht bevorzugt C₂-C₅-Alkyl sowie, gegebenenfalls durch NH₂, OH, C₁-C₄-Alkoxy oder Halogen substituiertes Phenyl oder Benzyl.

Die folgenden Beispiele dienen der Veranschaulichung der Erfindung.

### Beispiel 1:

### Racematspaltung von trans 2-Amino-Cyclohexancarbonsäuremethylester

50 g (0.32 mol) trans 2-Amino-Cyclohexancarbonsäuremethylester wurden in 200 ml MTBE gelöst, mit 0.16 mol Methoxyessigsäureisopropylester und 0.5 g Novozym 435 (Novo Nordisk) versetzt und 8 h bei Raumtemperatur geschüttelt. Nach Abfiltrieren des Enzyms wurde das Lösungsmittel entfernt und der Rückstand im Vakuum destilliert.
Man erhielt 23.5 g (1S,2S)-2-Amino-Cyclohexancarbonsäuremethylester und 35.2 g (1R,2R)-(N-2-Methoxy-acetyl)-2-Amino-Cyclohexancarbonsäuremethylester mit jeweils einem ee-Wert von 99.8%. Die ee-Bestimmung des Amins erfolgte nach N-Acylierung mit Acetanhydrid gaschromatographisch auf einer B-PH-GC-Säule (Fa. Astec, NJ, USA), das N-2-Methoxy-acetylamid wurde ohne Derivatisierung auf dieser Säule getrennt.

### Beispiel 2:

### Racematspaltung von Phenylalanin-methylester

88.4 g (0.5 mol) racemischer Phenylalaninmethylester wurden in 500 ml MTBE gelöst, mit 0.51 mol Methoxyessigsäureethylester und 0.8 g Novozym 435 (Novo Nordisk) versetzt und 8 h bei Raumtemperatur geschüttelt. Nach Abfiltrieren des Enzyms wurde der nicht acylierte (S)-Phenylalaninmethylester durch Extraktion mit 150 ml 2M HCl abgetrennt und anschließend aus der sauren wäßrigen Phase durch Behandlung mit 160 ml 2M NaOH und nachfolgender Extraktion mit 1 l Ethylacetat isoliert. Das acylierte Enantiomer wurde direkt aus der Reaktionslösung durch Abdampfen des Lösungsmittels isoliert.
Man erhielt 37.12 g (0.21 mol) (S)-Phenylalaninmethylester mit einem ee-Wert von 99% und 57.78 g (0.23 mol) des entsprechenden (N)-Methoxyacetamids des (R)-Phenylalaninmethylesters mit einem ee-Wert von 97.5%.

### Beispiel 3:

### Racematspaltung von 3-Aminobuttersäureethylester

13.1 g (0.1 mol) racemischer 3-Aminobuttersäureethylester wurden in 60 ml MTBE gelöst, mit 0.05 mol Methoxyessigsäureisopropylester und 130 mg Novozym 435 (Novo Nordisk) versetzt und 12 h bei Raumtemperatur geschüttelt. Nach Abfiltrieren des Enzyms wurde das Lösungsmittel entfernt und der Rückstand im Vakuum destilliert.
Man erhielt 5.37 g (0.041 mol) (3S)-3-Aminobuttersäureethylester mit einem ee-Wert von 98.5% und 9.10 g (0.045 mol) des entsprechenden (N)-Methoxacetamids des (3R)-3-Aminobuttersäureethylesters mit einem ee-Wert von >99%.

## Patentansprüche

1. Verfahren zur Herstellung von acylierten Aminosäureestern durch Umsetzung der Aminosäureester mit einem Acylierungsmittel in Gegenwart einer Hydrolase, ausgewählt aus der Gruppe Amidase, Protease, Esterase und Lipase, **dadurch gekennzeichnet, daß** das Acylierungsmittel ein Carbonsäurealkylester ist, dessen Säurekomponente ein Halogen-, Stickstoff-, Sauerstoffoder Schwefelatom trägt, das an ein Kohlenstoffatom in alpha-, beta- oder gamma-Position zum Carbonylkohlenstoff gebunden ist und dessen Alkoholkomponente einen unsubstituierten, verzweigt- oder geradkettigen C₁-C₁₀-Alkylrest darstellt.

2. Verfahren zur Herstellung von optisch aktiven Aminosäureestern aus racemischen Aminosäureestern, **dadurch gekennzeichnet, dass** man
a) einen racemischen Aminosäureester mit einem Carbonsäurealkylester als Acylierungsmittel, dessen Säurekomponente ein Halogen-, Stickstoff-, Sauerstoff- oder Schwefelatom trägt, das an ein Kohlenstoffatom in alpha-, beta- oder gamma-Position zum Carbonylkohlenstoff gebunden ist und dessen Alkoholkomponente einen unsubstituierten, verzweigt- oder geradkettigen C₁-C₁₀-Alkylrest darstellt, in Gegenwart einer Hydrolase, ausgewählt aus der Gruppe Amidase, Protease, Esterase und Lipase, enantioselektiv acyliert,
b) das Gemisch aus optisch aktivem Aminosäureester und optisch aktivem N-Acyl-Aminosäureester trennt und somit ein Enantiomer des Aminosäureesters erhält,
c) gegebenenfalls aus dem N-Acyl-Aminosäureester das andere Enantiomere des Aminosäureesters durch Amidspaltung gewinnt.

3. Verfahren gemäß Anspruch 1 und 2, **dadurch gekennzeichnet, dass** man einen Carbonsäurealkylester als Acylierungsmittel verwendet, dessen Säurekomponente in alpha-Position ein Halogen-, Stickstoff-, Schwefel- oder Sauerstoffatom trägt.

4. Verfahren gemäß Anspruch 1 und 2, **dadurch gekennzeichnet, dass** man einen Carbonsäurealkylester als Acylierungsmittel verwendet, dessen Säurekomponente in alpha-Position ein Sauerstoffatom trägt.

5. Verfahren gemäß den Ansprüchen 2 bis 4, **dadurch gekennzeichnet, dass** man in Anschluß an Schritt b) oder c) in einem weiteren Schritt das nicht-gewünschte Enantiomer racemisiert und dieses racemische Gemisch in das Verfahren gemäß Anspruch 2 zurückführt.

6. Verfahren zur Herstellung von optisch aktiven Aminosäureestern, **dadurch gekennzeichnet, daß** es mindestens als Teilschritt ein Verfahren nach den Ansprüchen 2 bis 5 beinhaltet.

## Claims

1. A process for preparing acylated amino esters by reacting the amino esters with an acylating agent in the presence of a hydrolase selected from the group of amidase, protease, esterase and lipase, wherein the acylating agent is an alkyl carboxylate whose acid component has a halogen, nitrogen, oxygen or sulfur atom and which is linked to a carbon atom in the position alpha, beta or gamma to the carbonyl carbon and whose alcohol component is an unsubstituted branched or straight-chain C₁-C₁₀-alkyl, radical.

2. A process for preparing optically active amino esters from racemic amino esters, which comprises
a) enantioselectively acylating a racemic amino ester with an alkyl carboxylate as acylating agent whose acid component has a halogen, nitrogen, oxygen or sulfur atom and which is linked to a carbon atom in the position alpha, beta or gamma to the carbonyl carbon and whose alcohol component is an unsubstituted branched or straight-chain C₁-C₁₀-alkyl radical, in the presence of a hydrolase selected from the group of amidase, protease, esterase and lipase,
b) separating the mixture of optically active amino ester and optically active N-acylamino ester and thus obtaining one enantiomer of the amino ester,
c) where appropriate obtaining the other enantiomer of the amino ester from the N-acylamino ester by amide cleavage.

3. A process as claimed in claims 1 and 2, wherein the acylating agent used is an alkyl carboxylate whose acid component has a halogen, nitrogen, sulfur or oxygen atom in the alpha position.

4. A process as claimed in claims 1 and 2, wherein the acylating agent used is an alkyl carboxylate whose acid component has an oxygen atom in the alpha position.

5. A process as claimed in claims 2 to 4, wherein, following step b) or c), the unwanted enantiomer is racemized in another step, and this racemic mixture is returned to the process as claimed in claim 2.

6. A process for preparing optically active amino esters, in which at least one step comprises a process as claimed in claims 2 to 5.

## Revendications

1. Procédé pour la préparation d'esters d'acide aminé acylés par réaction des esters d'acide aminé avec un agent d'acylation en présence d'une hydrolase, choisie dans le groupe des amidases, des protéases, des estérases et des lipases, **caractérisé par le fait que** l'agent d'acylation est un ester alkylique d'acide carboxylique dont le composant acide porte un atome d'halogène, d'azote, d'oxygène ou de soufre, qui est lié à un atome de carbone en position alpha, bêta ou gamma par rapport au carbone du carbonyle et dont le composant alcool représente un reste alkyle en C₁-C₁₀ non-substitué, ramifié ou en chaîne droite.

2. Procédé pour la préparation d'esters d'acide aminé optiquement actifs à partir d'esters d'acide aminé racémiques, **caractérisé par le fait que**
a) on acyle de manière énantiosélective un ester d'acide aminé racémique avec, en tant qu'agent d'acylation, un ester alkylique d'acide carboxylique dont le composant acide porte un atome d'halogène, d'azote, d'oxygène ou de soufre, qui est lié à un atome de carbone en position alpha, bêta ou gamma par rapport au carbone du carbonyle et dont le composant alcool représente un reste alkyle en C₁-C₁₀ non-substitué, ramifié ou en chaîne droite, en présence d'une hydrolase, choisie dans le groupe des amidases, des protéases, des estérases et des lipases,
b) on sépare le mélange d'ester d'acide aminé optiquement actif et d'ester d'acide aminé N-acylé optiquement actif, et on obtient ainsi un énantiomère de l'ester d'acide aminé,
c) éventuellement on obtient par coupure d'amide à partir de l'ester d'acide aminé N-acylé l'autre énantiomère de l'ester d'acide aminé.

3. Procédé selon la revendication 1 et 2, **caractérisé par le fait qu'**on utilise comme agent d'acylation un ester alkylique d'acide carboxylique dont le composant acide porte en position alpha un atome d'halogène, d'azote, de soufre ou d'oxygène.

4. Procédé selon la revendication 1 et 2, **caractérisé par le fait qu'**on utilise comme agent d'acylation un ester alkylique d'acide carboxylique dont le composant acide porte en position alpha un atome d'oxygène.

5. Procédé selon les revendications 2 à 4, **caractérisé par le fait qu'**on racémise à la suite de l'étape b) ou c), dans une autre étape, l'énantiomère non-souhaité, et on recycle ce mélange racémique dans le procédé selon la revendication 2.

6. Procédé pour la préparation d'esters d'acide aminé optiquement actifs, **caractérisé par le fait qu'**il comporte au moins comme étape partielle un procédé selon les revendications 2 à 5.
